(19) Europäisches Patentamt
European Patent Office
Office européen des brevets

(11) **EP 3 135 293 A1**

(12) **EUROPEAN PATENT APPLICATION**
published in accordance with Art. 153(4) EPC

(43) Date of publication:
01.03.2017 Bulletin 2017/09

(21) Application number: 15783227.0

(22) Date of filing: 20.03.2015

(51) Int Cl.:
*A61K 36/82* (2006.01)    *A61K 31/352* (2006.01)
*A61K 31/353* (2006.01)    *A61P 3/06* (2006.01)
*A61P 3/10* (2006.01)    *A61P 43/00* (2006.01)

(86) International application number:
PCT/JP2015/058488

(87) International publication number:
WO 2015/163062 (29.10.2015 Gazette 2015/43)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 23.04.2014 JP 2014089549

(71) Applicants:
• Nippon Paper Industries Co., Ltd.
Tokyo 114-0002 (JP)
• Kyushu University,
National University Corporation
Fukuoka-shi, Fukuoka 812-8581 (JP)

(72) Inventors:
• WASAI, Masafumi
Tokyo 114-0002 (JP)
• KAWAOKA, Akiyoshi
Tokyo 114-0002 (JP)
• TACHIBANA, Hirofumi
Fukuoka-shi
Fukuoka 812-8581 (JP)

(74) Representative: Müller-Boré & Partner
Patentanwälte PartG mbB
Friedenheimer Brücke 21
80639 München (DE)

(54) **AGENT FOR PREVENTING OR AMELIORATING DIABETES**

(57) An object of the present invention is to find a component that is safe and is capable of effectively exerting various effects such as prevention or amelioration of diabetes, prevention or amelioration of metabolic syndrome, amelioration of insulin resistance, inhibition of an increase in a postprandial blood sugar level, and inhibition of $\alpha$-glucosidase activity. The present invention provides agents for preventing or ameliorating diabetes or the like, the agents including a component of a hybrid plant of Camellia sinensis and Camellia taliensis as an active ingredient. The hybrid plant is preferably Sun Rouge, and each of the agents preferably includes a tea leaf extract of the hybrid plant.

FIG. 1

Data means ±S.E. , n=6 , Tukey's Test

## Description

TECHNICAL FIELD

The present invention relates to an agent for preventing or ameliorating diabetes, an agent for preventing or ameliorating metabolic syndrome, an agent for inhibiting or ameliorating an increase in HOMA-IR, an agent for inhibiting or ameliorating fat accumulation, an agent for inhibiting an increase in a postprandial blood sugar level, an agent for inhibiting glycolysis, an agent for inhibiting $\alpha$-glucosidase activity, an agent for inhibiting amylase activity, an agent for promoting lipid elimination, an agent for inhibiting lipase activity, and enhancing lipid metabolism.

BACKGROUND ART

Excess calorie intake has been a cause of lifestyle-related illnesses. Reducing digestion and absorption of carbohydrates including starches in particular is considered effective for prevention of and treatment for obesity.

Guava is a shrub native to Central America, and its fruits, roots and leaves are popularly used to treat diabetes and diarrhea as an herbal medicine. An extract extracted from its leaves in water or a hydrophilic solvent has been recently found to have an effect of inhibiting $\alpha$-amylase activity, and is therefore used as health drinks (Patent Literature 1) or diet foods and drinks (Patent Literature 2).

Furthermore, an indigestible dextrin, which is watersoluble dietary fiber, prepared by roasting corn starch, hydrolyzing it with amylase followed by taking out indigestible components has been found to have an effect of inhibiting an increase in a postprandial blood sugar level (Non Patent Literature 1), and used as an ingredient of foods for specified health uses.

On the other hands, it has been reported that for the component of the plant of Camellia sinensis, which is a tea tree, catechins have an effect of inhibiting an increase in a cholesterol level (Patent Literature 3), and non-polymeric catechins have various effects such as promoted combustion of accumulated body fats, promoted combustion of dietary fats, and promoted expression of a hepatic $\beta$-oxidation gene (Patent Literature 4).

PRIOR ART REFERENCE

Patent Literature

Patent Literature 1: Japanese Examined Patent Application Laid-open No. S60-36746
Patent Literature 2: Japanese Patent Application Laid-open No. H7-59539
Patent Literature 3: Japanese Patent Application Laid-open No. S60-156614
Patent Literature 4: Japanese Patent Application Laid-open No. 2002-326932

Non Patent Literature

Non Patent Literature 1: Journal of Nutritional Food, Vol. 2, No. 4, 1999

SUMMARY OF INVENTION

PROBLEM TO BE SOLVED BY THE INVENTION

However, a report in which the component of the plant of Camellia sinensis has effects related to prevention or amelioration of diabetes such as an effective action of inhibiting an increase in a postprandial blood sugar level has not been known. An object of the present invention is to find a component that is safe and is capable of effectively exerting various effects such as amelioration of insulin resistance, inhibition or amelioration of an increase in HOMA-IR, inhibition or amelioration of a worsening of hyperglycemia, inhibition of an increase in a postprandial blood sugar level, inhibition of $\alpha$-glucosidase activity, inhibition of amylase activity, inhibition of lipase activity, enhanced lipid metabolism, promoted lipid elimination, inhibition or amelioration of fat accumulation, and prevention or amelioration of diabetes.

MEANS FOR SOLVING PROBLEM

The present inventors have investigated the component or components of the plants of various related varieties of Camellia sinensis, and have found that a component or components of a hybrid plant of Camellia sinensis and its related variety that is Camellia taliensis have an effect of ameliorating insulin resistance, and also have an effect of inhibiting $\alpha$-glucosidase activity as well as an effect of inhibiting an increase in a blood sugar level such as postprandial blood sugar level. Furthermore, the present inventors have found that the component or components of the plant have an effect of inhibiting amylase activity, an effect of promoting lipid elimination, have an effect of inhibiting lipase activity and an effect of enhancing lipid metabolism, and have reached the present invention.

That is, the present invention is as follows.

[1] An agent for preventing or ameliorating diabetes, comprising a component of a hybrid plant of Camellia sinensis and Camellia taliensis as an active ingredient.

[2] An agent for preventing or ameliorating metabolic syndrome, comprising a component of a hybrid plant of Camellia sinensis and Camellia taliensis as an active ingredient.

[3] An agent for inhibiting or ameliorating an increase in HOMA-IR, comprising a component of a hybrid plant of Camellia sinensis and Camellia taliensis as an active ingredient.

[4] An agent for inhibiting or ameliorating fat accumulation, comprising a component of a hybrid plant of Camellia sinensis and Camellia taliensis as an active ingredient.

[5] An agent for inhibiting an increase in a postprandial blood sugar level, comprising a component of a hybrid plant of Camellia sinensis and Camellia taliensis as an active ingredient.

[6] An agent for inhibiting glycolysis, comprising a component of a hybrid plant of Camellia sinensis and Camellia taliensis as an active ingredient.

[7] An agent for inhibiting $\alpha$-glucosidase activity, comprising a component of a hybrid plant of Camellia sinensis and Camellia taliensis as an active ingredient.

[8] An agent for inhibiting amylase activity, comprising a component of a hybrid plant of Camellia sinensis and Camellia taliensis as an active ingredient.

[9] An agent for promoting lipid elimination, comprising a component of a hybrid plant of Camellia sinensis and Camellia taliensis as an active ingredient.

[10] An agent for inhibiting lipase activity and enhancing lipid metabolism, comprising a component of a hybrid plant of Camellia sinensis and Camellia taliensis as an active ingredient.

[11] The agent according to any one of [1] to [10], wherein the hybrid plant of Camellia sinensis and Camellia taliensis is Sun Rouge (Camellia sinensis (L.) Kuntze sp Sunrouge).

[12] The agent according to any one of [1] to [11], comprising a tea leaf extract of the hybrid plant of Camellia sinensis and Camellia taliensis.

[13] The agent according to [12], wherein the tea leaf extract comprises 0.13% by weight or more of delphinidin or its glycoside relative to the dry weight of the tea leaf extract.

[14] A method of ameliorating or preventing diabetes, comprising ameliorating or preventing diabetes of a subject by using an agent or a composition comprising a component of a hybrid plant of Camellia sinensis and Camellia taliensis as an active ingredient to the subject.

EFFECTS OF INVENTION

The present invention provides an agent for preventing or ameliorating diabetes, an agent for preventing or ameliorating metabolic syndrome, an agent for inhibiting or ameliorating an increase in HOMA-IR, an agent for inhibiting or ameliorating fat accumulation, an agent for inhibiting an increase in a postprandial blood sugar level, an agent for inhibiting glycolysis, an agent for inhibiting $\alpha$-glucosidase activity, an agent for inhibiting amylase activity, an agent for promoting lipid elimination, and an agent for inhibiting lipase activity and enhancing lipid metabolism. Each of the agents of the present invention includes a component or components of a hybrid plant of Camellia sinensis and Camellia taliensis, which have been conventionally consumed as tea, as an active ingredient: therefore, the agent is highly safe to ingest over a long period of time, and can be ingested daily.

BRIEF DESCRIPTION OF DRAWINGS

FIG. 1 is a graph illustrating the measurement results of the fat weight around the testicles of mice in test example 1.
FIG. 2 is a graph illustrating HOMA-IR in test example 1.
FIG. 3 is a graph illustrating AUC of postprandial blood sugar levels (from 0 minutes to 150 minutes) in test example 2.
FIG. 4 is a graph illustrating AUC of postprandial blood sugar levels (from 0 minutes to 30 minutes) in test example 2.
FIG. 5 is a graph illustrating the inhibition rates of $\alpha$-glucosidase activity in test example 3.
FIG. 6 is a graph illustrating AUC of postprandial blood sugar levels (from 0 minutes to 30 minutes) in test example 6.

FIG. 7 is a graph illustrating residual rates of delphinidin in test example 10.

EMBODIMENTS FOR CARRYING OUT THE INVENTION

The agents of the present invention include a component or components of a hybrid plant of Camellia sinensis and Camellia taliensis as an active ingredient.

The hybrid plant of Camellia sinensis and Camellia taliensis is a variety obtained by combining a plant that belongs to Camellia sinensis and a plant that belongs to Camellia taliensis and being propagated. The propagation may be artificial or natural such as self-pollination. The hybrid plant may be any of the first generation (the first filial generation) and its descendants (for example, a natural hybrid of the first filial generations). Camellia sinensis is also called as tea plant. Camellia sinensis is preferably a variety for green tea (Yabukita, Okumusashi, or the like) and more preferably Okumusashi. Camellia taliensis is a plant of Theaceae, is a related variety of Camellia sinensis, and is also called as akame.

The hybrid of Camellia taliensis and Camellia taliensis is preferably "Sun Rouge" (Camellia sinensis (L.) Kuntze, variety number 21262 registered with the Ministry of Agriculture, Forestry and Fisheries, Japan) and F95181 (Cha Chuukanbohon Nou-6), more preferably "Sun Rouge" as the hybrid.

Examples of the agents of the present invention include an agent for ameliorating insulin resistance, an agent for inhibiting glycolysis, an agent for inhibiting α-glucosidase, an agent for inhibiting lipase activity and enhancing lipid metabolism, an agent for inhibiting amylase activity, an agent for inhibiting an increase in a postprandial blood sugar level, an agent for promoting lipid elimination, an agent for inhibiting or ameliorating fat accumulation, an agent for preventing or ameliorating metabolic syndrome, an agent for inhibiting or ameliorating an increase in HOMA-IR, an agent for inhibiting or ameliorating a worsening of hyperglycemia, and an agent for preventing or ameliorating diabetes.

The agents of the present invention for preventing or ameliorating diabetes can prevent or ameliorate diabetes (usually, type II diabetes) and metabolic syndrome. Metabolic syndrome means a state in which visceral fat obesity as a common cause triggers hyperglycemia, dyslipidemia, and hypertension.

The prevention means inhibiting affection. Examples of preventing diabetes include preventing the index of blood sugar levels (fasting blood sugar level, postprandial blood sugar levels (blood sugar level 30 minutes after a meal, blood sugar level 2 hours after a meal, and the like), a blood sugar level in oral glucose tolerance test, hemoglobin (Hb) A1c values, and the like) of a subject from shifting to a diabetic state (for examples, preventing a normal state from shifting to a pre-diabetic state, maintaining a normal state, maintaining a pre-diabetic state, and shifting from a pre-diabetic state to a normal state).

The amelioration means that a person has already developed diabetes: however, the symptoms are improved, alleviated, or prevented from worsening. Specific examples include bringing the index of blood sugar levels of a subject being a diabetic state close to a normal value (for example, shifting the blood sugar levels to values of pre-diabetic state or normal state, improving the blood sugar levels close to a range of pre-diabetic state, but not yet getting out of a range of normal state).

Examples of prevention and amelioration of metabolic syndrome include normalization of dyslipidemia and normalization of blood pressure as well as the prevention and amelioration of diabetes mentioned above.

The confirmation of the effect of preventing or ameliorating diabetes may be done by one or more selected from confirmation of exerting an effect of ameliorating insulin resistance, confirmation of inhibition or amelioration of an increase in HOMA-IR, confirmation of exerting an effect of inhibiting an increase in a postprandial blood sugar level, confirmation of exerting an effect of inhibiting α-glucosidase activity, confirmation of exerting an effect of inhibiting amylase activity, confirmation of exerting an effect of inhibiting lipase activity, confirmation of exerting an effect of enhancing lipid metabolism, confirmation of exerting an effect of promoting lipid elimination, and confirmation of exerting an effect of inhibiting or ameliorating lipid accumulation.

The subject for the agent for preventing or ameliorating diabetes may be diabetics, potential diabetics, and persons having a risk of developing diabetes, and may also be healthy persons (for example, the aged, obese persons, persons with hypertension, and persons who want to prevent diabetes). The subject includes humans and animals other than humans (pets (for example, dogs, cats, and parakeets), domestic animals (for example, cattle, pigs, goats, sheep, and horses), fowls (for example, domestic fowls, quails, and turkeys), laboratory animals (for example, mice, rats, hamsters, guinea pigs, and rabbits)).

The agent of the present invention can ameliorate insulin resistance. Insulin resistance means being resistant to insulin. To ameliorate insulin resistance means that insulin resistance is made close to a normal value, or maintained in a range of a normal value. Insulin resistance may be checked by a method such as HOMA-IR (HOMA-R index, homeostasis model assessment ratio, homeostasis model assessment as an index of insulin resistance), glucose clamp technique, steady state plasma glucose (SSPG) method, or estimation from a fasting serum insulin level and a blood sugar level, and among them, the measurement by HOMA-IR is preferred. Amelioration of insulin resistance may also be confirmed by inhibition of an increase in HOMA-IR or keeping HOMA-IR low. Thus, the agent of the present invention can inhibit or ameliorate an increase in HOMA-IR. Amelioration of insulin resistance inhibits an increase in a blood sugar level: therefore, the amelioration of insulin resistance may be confirmed by the inhibition of an increase in a blood sugar level.

The agent of the present invention can inhibit an increase in a postprandial blood sugar level. Inhibition of an increase in a blood sugar level means that an increase in a blood sugar level is inhibited, or a blood sugar level is kept at a normal value. A blood sugar level is checked by measuring glucose in blood, and the confirmation of the inhibition of an increase in a blood sugar level is achieved by checking the measurement value of glucose in blood being within a range of a normal value.

Inhibition of an increase in a postprandial blood sugar level means that an increase in a postprandial blood sugar level is inhibited, or a postprandial blood sugar level is kept at a normal value. After a meal may be after finishing a meal; however, it is preferably 0 minutes to 30 minutes after finishing a meal. A postprandial blood sugar level is checked by area under the curve (AUC).
Amelioration of an increase in a blood sugar level is confirmed by a decrease in AUC.

The agent of the present invention can promote lipid elimination. Promotion of lipid elimination means that more amount of lipid is eliminated than usual. An amount of eliminated lipid is checked by measuring, for example, an amount of eliminated lipid in feces. Promoted lipid elimination is confirmed by more amount of lipid being eliminated in feces than usual. Furthermore, promoted lipid elimination helps to decrease body weight or decrease body fat; therefore, promotion of lipid elimination may be also confirmed by a decrease in body weight or a decrease in body fat.

As a result of promoted lipid elimination, the agent of the present invention inhibits a body weight increase, and exerts an anti-obesity action. Consequently, it is expected that the agent of the present invention inhibits or ameliorates a worsening of hyperglycemia and prevents or ameliorates diabetes.

The agent of the present invention can inhibit or ameliorate lipid accumulation. Inhibition or amelioration of lipid accumulation means decreasing the amount of lipid to be newly accumulated, and decreasing the amount of already accumulated lipid. Inhibition or amelioration of lipid accumulation may be confirmed by body fat mass being maintained, or body fat mass being decreased. Instead of body fat mass, inhibition or amelioration of lipid accumulation may be confirmed by body weight being maintained, or body weight being decreased. Inhibition or amelioration of lipid accumulation may also be confirmed by promoted lipid elimination.

As a result of inhibited or ameliorated lipid accumulation, the agent of the present invention inhibits a body weight increase, and exerts an anti-obesity action. Consequently, it is expected that the agent of the present invention inhibits or ameliorates a worsening of hyperglycemia, and prevents or ameliorates diabetes.

The agent of the present invention can inhibit glycolysis. Inhibition of glycolysis is usually achieved by inhibiting the activities of the enzymes involved in glycolysis. Examples of such enzymes include $\alpha$-glucosidases and amylases.

The agent of the present invention can inhibit $\alpha$-glucosidase activity. $\alpha$-glucosidase is an enzyme to break down $\alpha$-1,4-glucoside bonds, and is involved in glycolysis. Inhibition of $\alpha$-glucosidase activity can inhibit the absorption of sugars by preventing glycolysis; therefore, an increase in a blood sugar level can be inhibited. As a result of the inhibition of an increase in blood sugar level, a body weight increase is inhibited, and an anti-obesity action is exerted. Consequently, it is expected that the agent of the present invention inhibits or ameliorates a worsening of hyperglycemia, and prevents or ameliorates diabetes. Inhibition of the activity means that the activity of the enzyme is reduced or stopped. The type of the inhibition of the activity may be any of substrate inhibition, competitive inhibition, noncompetitive inhibition, and uncompetitive inhibition, or a combination of at least two of these inhibitions (mixed inhibition). $\alpha$-glucosidase activity can be confirmed by measuring an amount of the decomposition product after $\alpha$-glucosidases act on a substrate. When the amount of the decomposition product is decreased, the inhibition of the activity can be confirmed. $\alpha$-glucosidase activity may be measured by using a colorimetry kit such as the QuantiChrom (TM) $\alpha$-Glucosidase Assay Kit (manufactured by BioAssay Systems). The inhibition of $\alpha$-glucosidase activity inhibits an increase in a blood sugar level: therefore, the inhibition of $\alpha$-glucosidase activity may be confirmed by the inhibition of an increase in a blood sugar level.

The agent of the present invention can inhibit amylase activity. Amylase is an enzyme that hydrolyzes starch. Inhibition of amylase activity can inhibit the breakdown of starch into sugars within a body. As a result, the absorption of sugars in a body can be inhibited to inhibit an increase in a blood sugar level. As a result of the inhibition of an increase in blood sugar level, a body weight increase is inhibited, and an anti-obesity action is exerted. Consequently, it is expected that the agent of the present invention inhibits or ameliorates a worsening of hyperglycemia, and prevents or ameliorates diabetes.

Amylase activity can be measured by a conventionally known method, and, for example, the MaxDiscovery (TM) Amylase Assay Kit (manufactured by Bioo Scientific Corporation), or the like may be used.

The agent of the present invention can inhibit lipase activity. Lipases are enzymes that hydrolyze glycerol esters to separate fatty acids. Lipases may be derived from the organs of any living organisms, and the examples include a pancreatic lipase derived from the pancreas. The agent of the present invention can particularly inhibit pancreatic lipase activity. The inhibition of the lipase inhibits the breakdown of lipid and consequently inhibits the absorption of lipid in a body. As a result, the inhibition of the lipase promotes the elimination of lipid from a body. Such action inhibits an increase in body weight and exerts an anti-obesity action. Consequently, it is expected that the agent of the present invention inhibits or ameliorates a worsening of hyperglycemia, and prevents or ameliorates diabetes.

The agent of the present invention can enhance lipid metabolism. The enhancement of lipid metabolism inhibits lipid accumulation in a body and promotes the elimination of lipid from a body. Such action inhibits an increase in body weight and exerts an anti-obesity action. Consequently, it is expected that the agent of the present invention inhibits or ameliorates a worsening of hyperglycemia, and prevents or ameliorates diabetes. The enhanced lipid metabolism may be confirmed by, for example, checking an increase in the expression amount of a gene involved in a lipid metabolism pathway in the liver. The amount of gene expression may be analyzed by using, for example, microarray analysis (Whole Mouse Genome Oligo DNA Microarray Kit Ver. 2.0 manufactured by Agilent Technologies).

The subject for the agent of the present invention for inhibiting $\alpha$-glucosidase activity may be diabetics, patients with postprandial hyperglycemia, patients with impaired glucose tolerance, patients with hyperlipidemia, patients with hypertension, potential patients of these problems, and persons having a risk of these problems, or may also be healthy persons (for example, the aged, obese persons, persons who do not want to experience an increase in a postprandial blood sugar level, and persons who want to prevent the development of the diseases mentioned above). The subject includes humans and animals other than humans.

The subject for the agent of the present invention for inhibiting amylase activity may be diabetics, patients with postprandial hyperglycemia, patients with impaired glucose tolerance, patients with hyperlipidemia, patients with hypertension, potential patients of these problems, and persons having a risk of these problems, or may also be healthy persons (for example, the aged, obese persons, persons who do not want to experience an increase in a postprandial blood sugar level, and persons who want to prevent the development of the diseases mentioned above). The subject includes humans and animals other than humans.

The subject for the agent of the present invention for inhibiting an increase in a postprandial blood sugar level may be patients with postprandial hyperglycemia (may often be patients with impaired glucose tolerance, diabetics, and/or patients with arterial sclerosis), potential patients of postprandial hyperglycemia, and persons having a risk of developing postprandial hyperglycemia, or may also be healthy persons (for example, the aged, obese persons, and persons who want to prevent the development of postprandial hyperglycemia). The subject includes humans and animals other than humans.

The subject for the agent of the present invention for inhibiting or ameliorating lipid accumulation may be patients with metabolic syndrome, diabetics, patients with hyperlipidemia, and patients with hypertension, or may also be healthy persons. The subject includes humans and animals other than humans.

The subject for the agent of the present invention for preventing or ameliorating metabolic syndrome may be diabetics and patients with metabolic syndrome, or may also be healthy persons (for example, the aged, obese persons, and persons who want to prevent the development of metabolic syndrome). The subject includes humans and animals other than humans.

The subject for the agent for ameliorating insulin resistance and for the agent for inhibiting or ameliorating an increase in HOMA-IR of the present invention may be patients with insulin resistance (may often be patients with diabetes, hypertension, hyperlipidemia and/or metabolic syndrome), and persons having its risk, or may also be healthy persons (for example, the aged, obese persons, and persons who want to prevent the development of insulin resistance). The subject includes humans and animals other than humans.

The subject for the agent of the present invention for inhibiting or ameliorating a worsening of hyperglycemia may be diabetics, patients with impaired glucose tolerance, potential patients of these problems, and persons having a risk of these problems, or may also be healthy persons (for example, the aged, obese persons, persons who do not want to experience an increase in a blood sugar level, and persons who want to prevent the development of the diseases mentioned above). The subject includes humans and animals other than humans.

The subject for the agent of the present invention for inhibiting lipase activity and enhancing lipid metabolism may be patients with metabolic syndrome, diabetics, patients with hyperlipidemia, and patients with hypertension, or may also be healthy persons. The subject includes humans and animals other than humans.

The subject for the agent of the present invention for promoting lipid elimination may be patients with metabolic syndrome, diabetics, patients with hyperlipidemia, and patients with hypertension, or may also be healthy persons. The subject includes humans and animals other than humans.

The component of a plant may be a component which is included in at least one portion of the plant. Examples of the portion of the plant include roots, a stem, leaves, flowers, fruits, branches, seeds, germs, and the like. Among them, the portion is preferably a portion including a stem and/or leaves, more preferably a stem and/or leaves. A method of preparing the component of the hybrid plant of Camellia sinensis and Camellia taliensis is not limited to a particular method, and examples of the method include extraction from the plant, purification, and the like. The agents of the present invention preferably include the component of the hybrid plant of Camellia sinensis and Camellia taliensis as a leaf (tea leaf) extract of the plant.

The agents of the present invention include the component of the hybrid plant of Camellia sinensis and Camellia taliensis as an active ingredient. Examples of the component include anthocyanin, delphinidin, glycosides of delphinidin, catechins (for example, epigallocatechin gallate), caffeine, amino acids, amino acid salts, hydrolyzable tannins (for example, strictinin), theogallin, theogallin derivatives, and the like. The component may be one kind alone, or may also be a combination of two kinds or more.

In the present invention, the component of the hybrid plant of Camellia sinensis and Camellia taliensis preferably includes at least delphinidin or its glycosides, more preferably includes at least delphinidin and/or its glycosides, and catechins, preferably includes at least delphinidin and/or its glycosides, catechins, and one or more kinds selected from caffeine, amino acids, amino acid salts, hydrolyzable tannins, theogallin, and theogallin derivatives. In the case where the agents of the present invention include two or more kinds of components, the agents of the present invention may be referred to as compositions.

Delphinidin is an anthocyanidin that comprises anthocyanin. Anthocyanidin is classified into delphinidin, cyanidin, pelargonidin, auratinidin, luteolinidin, peonidin, malvidin, petunidin, europinidin, rosinidin, and the like. Delphinidin is 3,5,7-trihydroxy-2-(3,4,5-trihydroxyphenyl)-1-benzopyrylium ($C_{15}H_{11}O_7$). Delphinidin glycosides are compounds in which a hydrogen atom of one or more hydroxy groups included in delphinidin is substituted with other substituent. A substitution site is preferably position 3 or 6. Examples of other substituent include a group of sugars such as galactose, glucose, or the like from which one hydrogen atom is eliminated. Examples of the delphinidin glycosides include delphinidin-3-O-(6-trans-p-coumaroyl)-β-galactoside (DCGa), delphinidin-O-β-galactoside (D3Ga), and delphinidin-3-β-glucoside (D3G). The agent of the present invention may include delphinidin or one kind of its glycosides, or may include a combination of delphinidin and one kind of its glycosides, or a combination of delphinidin and two or more kinds of the glycosides.

The amount of delphinidin or its glycosides to be included in the agent of the present invention is preferably 0.13% by weight or more, more preferably 0.16% by weight or more, further more preferably 0.19% by weight or more relative to the dry weight of an active ingredient (a tea leaf extract in the case where the agent of the present invention includes the tea leaf extract). The upper limit is not limited to a particular value; however, it is normally 0.5% by weight or less.

In the case where the agent of the present invention includes anthocyanin other than delphinidin, the proportion of the amount of delphinidin or its glycosides per the amount of anthocyanin is preferably 50% by weight or more, more preferably 60% by weight or more. The upper limit is not limited to a particular value; however, it is normally 90% by weight or less.

The amount of anthocyanin means the amount of anthocyanin relative to the dry weight of an active ingredient (a tea leaf extract in the case where the agent of the present invention includes the tea leaf extract). The amount of delphinidin or its glycosides means the amount of delphinidin or its glycosides relative to the dry weight of the agent of the present invention.

The amount of delphinidin or its glycosides relative to the dry weight of the agent of the present invention may be measured by high performance liquid chromatography (HPLC). The amount of anthocyanin relative to the dry weight of the agent of the present invention may be measured by HPLC.

Catechins include catechin (C), gallocatechin (GC), catechin gallate (CG), gallocatechin gallate (GCG), epicatechin (EC), epigallocatechin (EGC), epicatechin gallate (ECG), epigallocatechin gallate(EGCG), epigallocatechin-3-(3"-O-methyl) gallate (EGCG3"Me), and epicatechin-3-(3"-O-methyl) gallate (ECG3"Me).

The amounts of catechins may be measured by HPLC or colorimetry.

Caffeine is a purine alkaloid having a purine ring. Caffeine is included in foods and drinks such as coffee, cola, green tea, black tea, oolong tea, cocoa, chocolate, and health drinks, and caffeine included in tea bind to tannins; therefore, its effects are inhibited. Thus, caffeine included in tea has a weak stimulant action and a moderate action.

The amount of caffeine included in the agents of the present invention is preferably 5.4% by weight or lower, more preferably 5.2% by weight or lower, further more preferably 5.1% by weight or lower relative to the dry weight of an active ingredient (a tea leaf extract in the case where the agents of the present invention include the tea leaf extract). The lower limit is not limited to a particular value; however, it is normally 2.5% by weight or more in the case where processing of lowering the amount of caffeine is not performed.

The amount of caffeine may be measured by HPLC.

Amino acids are compounds having both an amino group and a carboxyl group. Examples of the amino acids include theanine, glycine, arginine, lysine, alanine, glutamine, glutamic acid, histidine, threonine, asparagine, aspartic acid, phenylalanine, leucine, valine, tryptophan, proline, cysteine, serine, tyrosine, isoleucine, and methionine. Examples of amino acid salts include alkali metal salts, alkali earth metal salts, ammonium salts, inorganic acid salts, organic acid salts, and the like.

The amount of the amino acids or their salts included in the agents of the present invention is preferably 1.0% by weight or more, preferably 1.3% by weight or more, more preferably 1.5% by weight or more relative to the dry weight of an active ingredient (a tea leaf extract in the case where the agents of the present invention include the tea leaf extract). The upper limit is not limited to a particular value; however, it is normally 4.5% by weight or less.

The amount of the amino acids or their salts relative to the dry weight of the agent of the present invention may be measured by HPLC.

The agents of the present invention exert an antiallergic effect by including a hydrolyzable tannin. Examples of the hydrolyzable tannin include strictinin, and the like, and strictinin is preferred. Strictinin is 1,2-di-O-galloyl-4,6-O-(S)-hexahydroxydiphenoyl-β-D-glucopyranose.

The amount of the hydrolyzable tannin included in the agents of the present invention is preferably 1.7% by weight or lower, more preferably 1.5% by weight or lower, further more preferably 1.4% by weight or lower relative to the dry weight of an active ingredient (a tea leaf extract in the case where the agents of the present invention include the tea leaf extract). The lower limit is not limited to a particular value; however, it is normally 0.6% by weight or more.

The amount of the hydrolyzable tannin such as strictinin may be measured by HPLC.

An antiallergic effect can be obtained by including theogallin or its derivatives. Theogallin is a trihydroxybenzoic acid glycoside, and is also called as (1S)-1β,3β,4β-trihydroxy-5α-(galloyloxy)cyclohexanecarboxylic acid.

The amount of theogallin or its derivatives to be included is preferably 5.0% by weight or lower, more preferably 3.0% by weight or lower, further more preferably 1.6% by weight or lower relative to the dry weight of an active ingredient (a tea leaf extract in the case where the agents of the present invention include the tea leaf extract). The lower limit is not limited to a particular value; however, it is normally 0.7% by weight or more.

The amount of theogallin may be measured by HPLC.

The agents of the present invention preferably include the tea leaf extract of the hybrid plant of Camellia sinensis and Camellia taliensis, more preferably includes the tea leaf extract as an active ingredient. By doing so, the active ingredient mentioned above can be included with a balanced proportion and various effects can be exerted significantly.

The delphinidin or its glycosides included in the tea leaf extract have higher stability than a conventionally known anthocyanin. The stability can be expressed by the residual rate of delphinidin or its glycosides in the tea leaf extract when the tea leaf extract is heated at the temperature of 100 °C for the period of 120 minutes. The residual rate is preferably 90% by weight or more. The residual rate of delphinidin or its glycosides is calculated by a formula (1).

```
Formula (1):

Residual rate of delphinidin or its glycosides (%) = {(the

amount of delphinidin or its glycosides when the tea leaf extract is heated at the temperature of 100 °C for the period of 120 minutes) / (the amount of delphinidin or its glycosides in the tea leaf extract before being heated under the above conditions)} × 100
```

The conditions of the extraction of the tea leaf extract do not matter in particular. For example, the tea leaf extract is obtained by being extracted from the tea leaves of the hybrid plant of Camellia sinensis and Camellia taliensis.

The tea leaves are usually processed before the extraction. Examples of the processed tea leaves include green tea, black tea, oolong tea and the like, and green tea is preferred. The examples of the tea leaves of green tea include Sencha, Gyokuro, Bancha, Kukicha, Mecha, Konacha, Houjicha, Genmaicha, Tencha, Kamairicha, Hoshucha, and the like.

The tea leaves may be partially ground before the extraction, and grinding is preferably performed. The particle diameter of the powder is preferably 10 mm or less, more preferably 5 mm or less, further more preferably 1 mm or less. The grinding is done by using a grinder (for example, the multi-beads shocker (TM, Yasui Kikai Corporation), a stone mill, a ceramic mill, a ball mill, a hammer mill, or the like.

An extraction solvent may be water or an ethanol aqueous solution, and water is preferred. The concentration of ethanol in the ethanol aqueous solution may be set within a range that does not impair the effects of the present invention, and usually is 70% by weight or lower relative to the aqueous ethanol solution. In case of water extraction, the extraction temperature is preferably 70 °C to 100 °C, more preferably 70 °C to 80 °C. The extraction period is preferably 5 minutes to 60 minutes, more preferably 5 minutes to 10 minutes. For the weight ratio of water and the tea leaves, water in an amount of 10 times or more with respect to 1 dry weight of the tea leaves is preferably used, and water in an amount of 10 times to 100 times more than the dry weight of the tea leaves is more preferably used. If the extraction temperature is more than 100 °C and the extraction period is longer than 60 minutes, the amounts of catechins increase; therefore, the bitterness and the astringency become stronger and the taste might be deteriorated. In case of the extraction by an organic solvent such as acetic acid, the extraction efficiency improves, but the amounts of catechins increase;

therefore, the bitterness and the astringency become stronger and the taste might be deteriorated. Stirring may be performed at the time of extraction.

The tea leaf extract is preferably a tea leaf extract obtained by being extracted at a temperature of 70 °C to 100 °C and for a period of 5 minutes to 60 minutes in water in an amount 10 times or more than the dry weight of the tea leaves.

Solids may be removed by a treatment such as filtration, centrifugation, or sifting after the extraction (solid-liquid separation). The filtration may be performed once, or twice or more. For the filtration, a unit such as filter paper or a line filter may be used. The centrifugation may be performed at 100 g to 15,000 g for a period of 1 minute to 60 minutes. Furthermore, treatments such as concentration (vacuum concentration, reverse osmosis membrane treatment, or the like) and drying (spray drying, freeze-drying, or the like) may be performed, and performing both concentration and drying is preferred. The treatments are preferably performed such that the concentrate has an amount 0.05 times of the weight of the extract before the treatments, and the dried product has an amount 0.2 times of the weight of tea leaves. Filtration or sifting may be performed after concentration and/or drying.

The agents of the present invention are not limited to a particular form. Examples of the form include liquid (capsules, soft gelatin capsules, and the like), slurries (syrup, and the like), semisolids (cream, paste, and the like), and solids (tablets, powder (granules, finer granules, and the like)), solids are preferred, and powder is preferred. The dosage form of the agent of the present invention is not limited. Examples of the dosage form include pills, tablets, granules, suspensions, emulsions, powders, syrups, and troches.

The agents of the present invention are not limited to a particular administration form. Examples include oral administration, parenteral administration (pulmonary administration, subcutaneous administration, intravenous administration, intrathecal administration, dermal administration, and the like), sublingual administration, inhalation administration, and the like, and oral administration is usually practiced.

The agents of the present invention may include a component other than the active ingredient. In the case where the agents of the present invention include a component other than the active ingredient, the agents of the present invention may be referred to as a composition. Examples of the components other than the active ingredient include various additives. Specific examples of the additives include the followings: seasonings; acidulants (citric acid, succinic acid, and the like); preservatives (ascorbic acid, acetates, ε-polylysine, and the like); pH adjusting agents; emulsifiers (saccharose fatty esters, glycerin fatty acid esters, organic acid monoglycerides, lecithin, and the like); aromatics; pigments; thickeners (carrageenan, xanthan gum, and the like); leavening agents; proteins (soy proteins, dairy proteins, and the like); saccharides (starches, sucrose, fructose, hydrogenated starch hydrolysates, erythritol, xylitol, and the like); sweeteners (sucralose, thaumatin, and the like); vitamins (vitamin A, vitamin C, vitamin E, vitamin K, and the like); and minerals (iron, calcium, and the like).

The agents of the present invention may be used as foods and drinks, or additives for foods and drinks. Examples of the foods and drinks include drinks (soft drinks, carbonated drinks, nutrient drinks, powdered drink mixes, fruit drinks, lactic drinks, jelly drinks, beers, sakes, foreign liquors, Chinese liquors, alcoholic medicinal drinks, and the like); rice (rice, rice gruel, and the like); breads; seasonings (sauces, misos, soy sauces, mayonnaises, shortenings, dressings, dipping sauces, spices, and the like); soybean foods (fermented soybeans, tofu, deep-fried tofu, and the like); processed marine products (kamaboko, hanpen, chikuwa, fish cakes, and the like); processed meat products (hams, sausages, Vienna sausages, and the like); processed agricultural foods (vegetables, fruits, and the like); pickles; noodles (udon, buckwheat noodles, spaghetti, and the like); soups (powdered soups, liquid soups, and the like); dairy products (cheeses, yogurts, creams, and the like); snacks (jellies, snack foods, chewing gums, candies, chocolates, cakes and the like); health foods (functional foods, dietary supplements (supplements), and foods for specified health uses); and the foods and drinks that specify subjects (foods for medical use, foods for patients, baby foods, nursing meals, and foods for the aged), and the like. Examples of foods and drinks for the additives for foods and drinks are similar to the specific examples mentioned above. Furthermore, the agents of the present invention can be used as feeds or additives for feeds. The subject for the feeds may be animals other than humans.

The administration time of the agents of the present invention is not limited to a particular time; however, in case of the agent for inhibiting an increase in a postprandial blood sugar level, the agent for inhibiting glycolysis, the agent for inhibiting α-glucosidase activity, and the agent for inhibiting amylase activity, the agents of the present invention are preferably administered before a meal (for example, from 30 minutes before a meal to the start of a meal), during a meal, or after a meal (for example from the end of a meal to 30 minutes after the end of a meal), and more preferably administered during a meal.

The dose of the agents of the present invention is not limited to a particular amount, and can be properly adjusted depending on the age, weight, health condition, and the like of the subject of the administration. A daily dose of the agents of the present invention is, as tea leaves, preferably 0.5 g or more, more preferably 1.0 g or more, further more preferably 1.5 g or more. There is no upper limit for a daily dose of the agents of the present invention; however, it is, as tea leaves, normally 50 g or less.

The agents of the present invention (the compositions, in the case where the agents of the present invention include two or more components) are used for a subject, and can ameliorate or prevent diabetes of the subject. Accordingly, a method of using the agents or compositions of the present invention for a subject to ameliorate or prevent diabetes of the subject is understood by the present specification. Examples and preferred examples of the scope of the subject, the agents or compositions which can be used, the preferred dosage of the agents or compositions which can be used, the preferred dosage time, and the administration form of said method are the same as the examples described for the agents of the present invention.

EXAMPLES

Test example 1 Evaluation of the prevention of metabolic syndrome

[Example 1]

For tea leaves, Sun Rouge dry tea leaves (unprocessed tea leaves) obtained by picking "Sun Rouge" tea leaves of the year 2012, and then steaming and drying the leaves were used.

An extract was obtained by reducing the size of the unprocessed Sun Rouge tea leaves into approximately 5 mm by a mill (manufactured by Tiger Corporation), adding hot water of 70 °C in an amount 20 times more (w/w) of the reduced raw material, stirring the mixture at 70 °C $\pm$ 2 °C for 10 minutes, and then separating the mixture into solid and liquid with a 100 mesh. A Sun Rouge tea leaf extract sample in powder form was obtained by centrifuging the extract at 700 g for 10 minutes, collecting supernatant liquid, and then processing and freeze-drying the liquid by the vacuum freeze dryer (model FZ-12CS, BTD shelf type drying chamber manufactured by LABCONCO).

1% by weight of the resulting Sun Rouge tea leaf extract sample was mixed into a feed. A feed composition is illustrated in Table 1.

[Table 1]

Table 1 Feed compositions in test example 1

| | Example 1 | Comparative Example 1 | Comparative Example 2 |
|---|---|---|---|
| Ingredient (g/kg) | | | |
| Vitamin mix | 10 | 10 | 10 |
| Mineral mix | 35 | 35 | 35 |
| Choline bitartrate | 2.5 | 2.5 | 2.5 |
| L-Cystein | 3.8 | 3.8 | 3.8 |
| Soybean oil | 20 | 20 | 20 |
| Tertiary butylhydroquinone | 0.06 | 0.06 | 0.06 |
| Sucrose | 200 | 200 | 200 |
| Casein | 250 | 250 | 250 |
| Corn strach | 148.7 | 148.7 | 148.7 |
| Cellulose | 40 | 40 | 50 |
| Tallow | 140 | 140 | 140 |
| Lard | 140 | 140 | 140 |
| Yabukita extract powder | 0 | 10 | 0 |
| Sunrouge extract powder | 10 | 0 | 0 |
| Protein,% of energy | 21.6 | 21.6 | 21.6 |
| Fat,% of energy | 30.3 | 30.3 | 30.3 |
| Energy, MJ/kg | 21.1 | 21.1 | 21.1 |
| Energy, kcal/kg | 5039.5 | 5039.5 | 5039.5 |

The composition of the Sun Rouge tea leaf extract sample obtained in Example 1 was checked, and was the following. The amounts in the following are the amount relative to the dry weight of tea leaf extract.

[Composition of the Sun Rouge tea leaf extract sample]

- Anthocyanin 0.29% by weight

- Delphinidin 0.19% by weight

- Delphinidin / Anthocyanin ratio 0.68

- Catechins 23.1% by weight

- EGCG 12.0% by weight

- Caffeine 5.1% by weight

- Amino acids 1.5% by weight

- Strictinin 1.4% by weight

- Theogallin 1.5% by weight

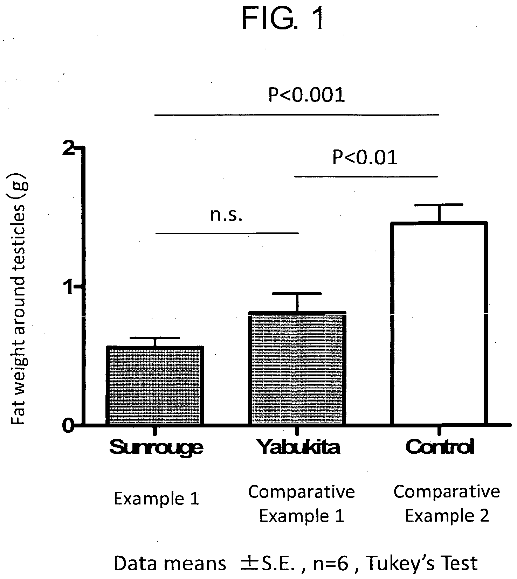

For the administration group of the Sun Rouge tea leaf extract sample (n = 6), male mice C57BL/6J at the age of 12 weeks were used. After preliminary breeding the mice of the administration group for 7 days, the group was bred for 8 weeks while the mice were freely fed such that the feed of each of the mice to which the Sun Rouge tea leaf extract sample was added was in an amount of 4 g per day. After the breeding period, blood was collected after 16 hours of fasting. After the blood collection, each of the mice was dissected to measure the fat weight around the testicles. The measurement results of the fat weight around the testicles of the mice are illustrated in FIG. 1.

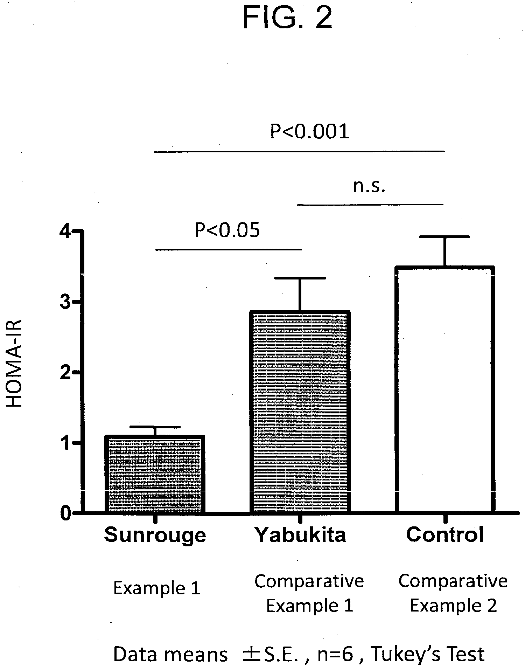

Blood serum was prepared from the collected blood, .and the obtained blood serum was analyzed by the Glucose CII-test Wako (manufactured by Wako Pure Chemical Industries, Ltd.) to measure a blood sugar level. Additionally, the obtained blood serum was analyzed by the ultrasensitive mouse Insulin Measurement Kit (manufactured by Morinaga Institute of Biological Science, Inc.) to measure an insulin level. HOMA-IR (the formula: {fasting blood insulin concentration ($\mu$U/mL) $\times$ fasting blood sugar level (mg/dl)} / 405) was calculated from the obtained data. The result is illustrated in FIG. 2.

[Comparative Example 1]

Comparative Example 1 was carried out in the same manner as Example 1 except that dried Yabukita tea leaves obtained by picking "Yabukita" tea leaves of the year 2013, and then steaming and drying the leaves were used in place of the dried Sun Rouge tea leaves. A feed composition is illustrated in Table 1, and the results are illustrated in FIG. 1 and FIG. 2.

[Comparative Example 2]

Comparative Example 2 was carried out in the same manner as Example 1 except that no tea leaf extract was added to a feed. A feed composition is illustrated in Table 1, and the results are illustrated in FIG. 1 and FIG. 2.

As illustrated in FIG. 1, the feed of Example 1 to which the Sun Rouge tea leaf extract sample was added has less fat weight than Comparative Example 1 in which the Yabukita tea leaf extract sample was added and Comparative Example 2 in which no tea leaf extract sample was added.

As illustrated in FIG. 2, the feed of Example 1 to which the Sun Rouge tea leaf extract sample was added significantly inhibited an increase in HOMA-IR compared to Comparative Example 1 in which the Yabukita tea leaf extract sample was added and Comparative Example 2 in which no tea leaf extract sample was added. These results demonstrate that the agent of the present invention can exert an effect of ameliorating insulin resistance.

Each of obesity and insulin resistance is a symptom of metabolic syndrome, and the results of test example 1 demonstrate that the agent of the present invention can exert an effect of preventing or ameliorating metabolic syndrome.

Text example 2 Evaluation of the inhibition of an increase in postprandial blood sugar levels.

[Example 2]

For the administration group of the Sun Rouge tea leaf extract sample (n = 6), male ICR mice at the age of 6 weeks were used. After preliminary breeding each of the mice for 3 weeks, blood was collected after 16 hours of fasting. Then, a Sun Rouge tea leaf extract sample similar to the one in Example 1 and soluble starch (manufactured by Wako Pure Chemical Industries, Ltd.) were mixed such that the amounts were 500 mg/kg of mouse weight and 2000 mg/kg of mouse weight respectively, and the mixture was administered into the stomach of each of the mice by a sonde.

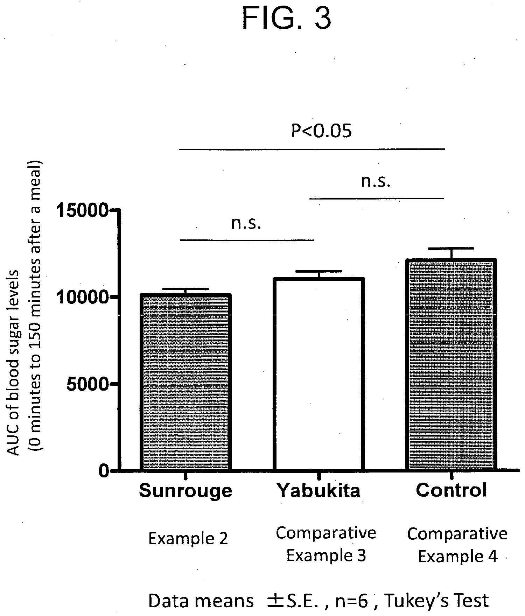

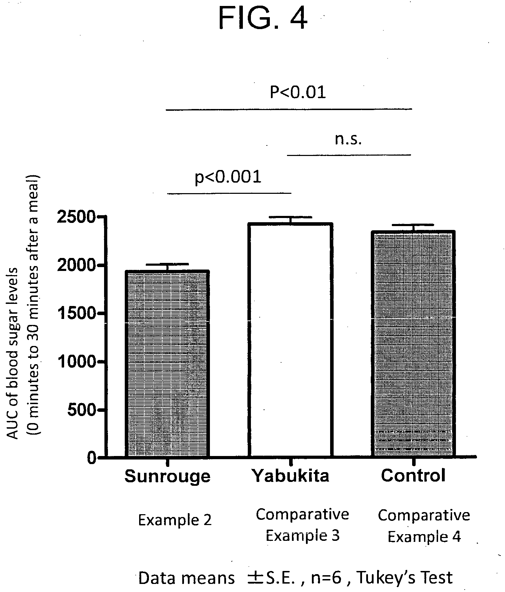

Blood was collected from each of the mice 30 minutes, 60 minutes, 120 minutes and 150 minutes after the administration of the tea leaf extract sample and the soluble starch to measure blood sugar levels by using the Medisafe Mini (manufactured by TERUMO CORPORATION) and the Medisafe Chip (manufactured by TERUMO CORPORATION). The AUC of postprandial blood sugar levels from 0 minutes to 150 minutes was calculated. The result is illustrated in FIG. 3. Furthermore, the AUC of postprandial blood sugar levels from 0 minutes to 30 minutes was calculated. The result is illustrated in FIG. 4.

[Comparative Example 3]

Comparative Example 3 was carried out in the same manner as Example 2 except that a Yabukita tea leaf extract sample similar to the one in Comparative Example 1 was used in place of the Sun Rouge tea leaf extract sample. The results are illustrated in FIG. 3 and FIG. 4.

[Comparative Example 4]

Comparative Example 4 was carried out in the same manner as Example 2 except that saline was used in place of the Sun Rouge tea leaf extract sample. The results are illustrated in FIG. 3 and FIG. 4.

As illustrated in FIG. 3, for the AUC from the start of a meal to 150 minutes after the start of the meal, the AUC of Example 2 in which the Sun Rouge tea leaf extract sample was administered was significantly lower than the AUC of Comparative Example 4 in which saline was administered. In the meantime, the AUC of Comparative Example 3 in which the Yabuki tea leaf extract sample was administered was slightly lower than the AUC of Comparative Example 4, and no significant difference was seen.

As illustrated in FIG. 4, for the AUC from the start of a meal to 30 minutes after the start of the meal, the AUC of Example 2 was significantly lower than the AUC of Comparative Example 3 and the AUC of Comparative Example 4. The AUC of Comparative Example 3 was rather higher than the AUC of Comparative Example 4.

These results demonstrate that the agent of the present invention can exert an effect of inhibiting an increase in blood sugar levels such as postprandial blood sugar levels.

Test example 3 The evaluation of the inhibition rate of $\alpha$-glucosidase activity (case 1)

[Example 3]

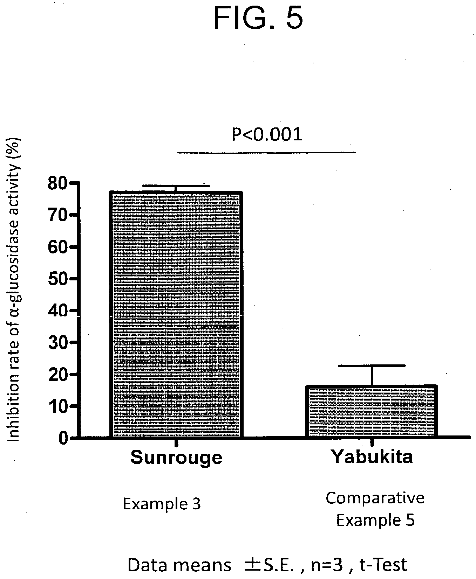

A Sun Rouge tea leaf extract sample similar to the one in Example 1 was diluted such that the final concentration was 100 mg/L. $\alpha$-glucosidase (manufactured by Wako Pure Chemical Industries, Ltd.) was diluted such that the final concentration was 100 U/L. The inhibition rate of the $\alpha$-glucosidase activity of the Sun Rouge tea leaf extract sample was measured by using each of the resulting diluted solutions and the QuantiChrom (TM) $\alpha$-Glucosidase Assay Kit (manufactured by BioAssay Systems). The result is illustrated in FIG. 5.

[Comparative Example 5]

Comparative Example 5 was carried out in the same manner as Example 3 except that a Yabukita tea leaf extract sample similar to the one in Comparative Example 1 was used in place of the Sun Rouge tea leaf extract sample. The result is illustrated in FIG. 5.

As illustrated in FIG. 5, Example 3 in which the Sun Rouge tea leaf extract sample was used showed a significantly higher effect of inhibiting the $\alpha$-glucosidase activity than Comparative Example 5 in which the Yabukita tea leaf extract sample was used. This result illustrates that the agent of the present invention can exert an effect of inhibiting an $\alpha$-glucosidase activity.

Test example 4 The evaluation of the inhibition rate of $\alpha$-glucosidase activity (case 2)

[Example 4]

1.0 mL of distilled water was added to 20 mg of dried Sun Rouge tea leaf powder to extract components at the temperature of 100 °C for 10 minutes. The mixture was centrifuged at 1700 g for 10 minutes, and then filtered using a 0.2 μm filter (manufactured by Whatman) to obtain an extract of the dried Sun Rouge tea leaves. The dried tea leaf powder was prepared by pulverizing unprocessed tea leaves by the multi-beads shocker (manufactured by Yasui Kikai Corporation).

α-glucosidase (manufactured by Wako Pure Chemical Industries, Ltd.) was diluted such that the final concentration was 100 U/L. The inhibition rate of the α-glucosidase activity of the Sun Rouge tea leaf extract sample was measured by using the resulting extracted sample and diluted solution, and the QuantiChrom (TM) α-Glucosidase Assay Kit (manufactured by BioAssay Systems). The result is illustrated in Table 2.

[Example 5]

Example 5 was carried out in the same manner as Example 4 except that dried tea leaves of Cha Chuukanbohon Nou-6 that is the mother plant of Sun Rouge were used in place of the dried Sun Rouge tea leaves. The result is illustrated in Table 2.

[Comparative Example 6]

Comparative Example 6 was carried out in the same manner as Example 4 except that each of 50 kinds of the dried tea leaves illustrated in Table 2 was used in place of the dried Sun Rouge tea leaves. The results are illustrated in Table 2.

[Table 2]

Table 2 Inhibition rates of α-glucosidase activity of tea leaf extract samples

|  | Varieties | Inhibition rates of α-glucosidase activity |
|---|---|---|
| Example 4 | Sun Rouge | 73.1% |
| Example 5 | Cha Chuukanbohon Nou-6 | 77.4% |
|  | Seishin oolong | 40.0% |
|  | Fukumidori | 14.3% |
|  | Benifuji | 29.2% |
|  | Minekaori | 19.1% |
|  | Benihikari | 32.2% |
|  | Minamikaori | 6.4% |
|  | Benihomare | 35.2% |
|  | Izumi | 21.7% |
|  | Fuushun | 5.3% |
|  | Tamamidori | 19.5% |
|  | Yamakai | 21.6% |
|  | Okumusashi | 22.0% |
|  | Dah Yeh Oolong | 25.0% |
|  | Seishintaipan | 13.6% |
|  | Kuritawase | 13.0% |
|  | Syunmei | 10.3% |
|  | Sayamamidori | 15.4% |
|  | Asagiri | 23.1% |
|  | Hokumei | 11.5% |
|  | Asahi | 24.5% |
|  | Sayamakaori | 18.5% |

(continued)

| | Varieties | Inhibition rates of $\alpha$-glucosidase activity |
|---|---|---|
| | Meiryoku | 18.8% |
| | Kanayamidori | 21.3% |
| Comparative Example 6 | Yamatomidori | 32.4% |
| | Asatsuyu | 21.7% |
| | Toyoka | 27.4% |
| | Yaeho | 23.9% |
| | Ujihikari | 25.7% |
| | Ooiwase | 18.8% |
| | Gokou | 16.0% |
| | Surugawase | 20.9% |
| | Samidori | 13.1% |
| | Komakage | 27.2% |
| | Hatsumomiji | 36.7% |
| | Ryoufuu | 15.8% |
| | Minamisayaka | 24.9% |
| | Saemidori | 10.6% |
| | Okuyutaka | 17.5% |
| | Okumidori | 9.2% |
| | Yutakamidori | 17.4% |
| | Yabukita | 30.3% |
| | Benifuuki | 48.8% |
| | Benibana | 42.5% |
| | Harumoegi | 16.3% |
| | Yumewakaba | 17.0% |
| | Yumekaori | 17.0% |
| | Sohu | 13.3% |
| | Sakimidori | 4.0% |
| | Harumidori | 18.7% |
| | Sainomidori | 28.5% |

As illustrated in FIG. 2, Example 4 in which the extract sample of the dried Sun Rouge tea leaf powder was used and Example 5 in which the Cha Chuukanbohon Nou-6 tea leaf powder extract sample was used showed higher effects of inhibiting the $\alpha$-glucosidase activity than Comparative Example 6 in which other tea leaf powder extract samples were used.

These results demonstrate that the agent of the present invention can stably exert an effect of inhibiting $\alpha$-glucosidase activity without being affected by extraction conditions or the like.

The results of Examples mentioned above demonstrate that the agents of the present invention can inhibit an increase in postprandial blood levels as a result of exerting an effect of inhibiting $\alpha$-glucosidase activity, consequently can prevent or ameliorate obesity to inhibit a worsening of hyperglycemia or to ameliorate hyperglycemia, and can exert an effect of preventing or inhibiting diabetes.

Test example 5 The evaluation of the inhibition of amylase activity

[Example 6]

1.0 mL of distilled water was added to 20 mg of dried Sun Rouge tea leaf powder to extract components at the temperature of 100 °C for 10 minutes. The mixture was centrifuged at 1700 g for 10 minutes, and then filtered using a 0.2 μm filter (manufactured by Whatman) to obtain an extract of the dried Sun Rouge tea leaves. The dried tea leaf powder was prepared by pulverizing unprocessed tea leaves by the multi-beads shocker (manufactured by Yasui Kikai Corporation).

An amylase solution was obtained by adjusting the final concentration of the amylase derived from pig pancreas (manufactured by Sigma-Aldrich Co. LLC) to be 800 U/L. "A: the amylase activity of the mixed solution of the extract of the dried Sun Rouge tea leaves and the amylase solution" and "B: "the amylase activity of the amylase solution" were respectively measured by the MaxDiscovery (TM) Amylase Assay Kit (manufactured by Bioo Scientific Corporation), and the inhibition rate of the amylase activity of the extract of the dried Sun Rouge tea leaves was calculated by dividing A by B. The results are illustrated in Table 3.

[Example 7]

Example 7 was carried out in the same manner as Example 6 except that dried tea leaves of Cha Chuukanbohon Nou-6 were used in place of the dried Sun Rouge tea leaves.

[Comparative Example 7]

Comparative Example 7 was carried out in the same manner as Example 6 except that 50 kinds of the tea leaves illustrated in Table 3 were used in place of the dried Sun Rouge tea leaves. The results are illustrated in Table 3.

[Table 3]

Table 3 Inhibition rates of amylase activity of tea leaf extract samples

|  |  | Varieties | inhibition rates of amylase activity |
|---|---|---|---|
| Example 6 |  | Sun Rouge | 45.2% |
| Example 7 |  | Cha Chuukanbohon Nou-6 | 25.8% |
|  |  | Seishin oolong | 9.8% |
|  |  | Fukumidori | 5.9% |
|  |  | Benifuji | 4.2% |
|  |  | Minekaori | 4.6% |
|  |  | Benihikari | 6.7% |
|  |  | Minamikaori | 8.9% |
|  |  | Benihomare | 16.6% |
|  |  | Izumi | 9.8% |
|  |  | Fuushun | 5.0% |
|  |  | Tamamidori | 5.6% |
|  |  | Yamakai | 8.0% |
|  |  | Okumusashi | 11.9% |
|  |  | Dah Yeh Oolong | 9.1% |
|  |  | Seishintaipan | 3.7% |
|  |  | Kuritawase | 5.6% |
|  |  | Syunmei | 9.3% |
|  |  | Sayamamidori | 5.6% |
|  |  | Asagiri | -4.6% |
|  |  | Hokumei | 2.4% |

(continued)

|  | Varieties | inhibition rates of amylase activity |
|---|---|---|
| Comparative Example 7 | Asahi | 2.6% |
| | Sayamakaori | 4.5% |
| | Meiryoku | 5.2% |
| | Kanayamidori | 16.9% |
| | Yamatomidori | 15.3% |
| | Asatsuyu | 12.9% |
| | Toyoka | 14.3% |
| | Yaeho | 1.3% |
| | Ujihikari | 21.0% |
| | Ooiwase | -7.4% |
| | Gokou | 23.1% |
| | Surugawase | 4.2% |
| | Samidori | 8.4% |
| | Komakage | -3.6% |
| | Hatsumomiji | 15.4% |
| | Ryoufuu | 3.0% |
| | Minamisayaka | 9.3% |
| | Saemidori | 5.6% |
| | Okuyutaka | 9.1% |
| | Okumidori | -1.2% |
| | Yutakamidori | 25.1% |
| | Yabukita | 9.7% |
| | Benifuuki | 11.6% |
| | Benibana | 16.8% |
| | Harumoegi | -17.6% |
| | Yumewakaba | 8.0% |
| | Yumekaori | 15.8% |
| | Sohu | 7.5% |
| | Sakimidori | 7.0% |
| | Harumidori | -3.0% |
| | Sainomidori | 3.6% |

As illustrated in FIG. 3, Example 6 in which the extract sample of the dried Sun Rouge tea leaf powder was used and Example 7 in which the extract sample of the dried Cha Chuukanbohon Nou-6 tea leaf powder was used showed higher effects of inhibiting the amylase activity than Comparative Example 7 in which the extract samples of other dried tea leaf powder were used. Particularly, Example 6 in which the extract sample of the dried Sun Rouge tea leaf powder was used showed a significantly high effect of inhibiting the amylase activity.

Text example 8 Evaluation of the inhibition of an increase in postprandial blood sugar levels in humans

[Example 8]

As mentioned below, postprandial blood sugar levels in humans were measured according to the protocol of Japanese Association for the Study of Glycemic Index.

6 persons without impaired glucose tolerance were chosen as the subjects. The subjects were prohibited from doing extreme exercises and eating a meal after 8 o'clock in the evening the day before the date of blood sugar level measurements. The subjects were also prohibited from excessive drinking and eating, drinking too much alcohol, and staying up till late the day before the date of blood sugar level measurements.

The subjects were prohibited from having breakfast on the day of blood sugar level measurements, and the measurements of blood sugar levels were started from 9 o'clock in the morning. Blood was collected from the fingertip of each of the subjects, and the blood sugar level was measured by the Medisafe blood sugar measurement kit (manufactured by TERUMO CORPORATION) to be a fasting blood sugar level.

Then, each of the subjects was subject to ingest the evaluation sample of 0.5 g of dried Sun Rouge tea leaf powder (produced in Tokushima in the year 2014, supplied by NIPPON PAPER INDUSTRIES CO., LTD.) along with 50 mL of distilled water. The dried Sun Rouge tea leaf powder was obtained by pulverizing unprocessed tea leaves by a mill (manufactured by Tiger Corporation). Each of the subjects was subject to ingest 130 g of rice (packed rice, Sato No Gohan, 1 serving, Koshihikari made in Niigata, manufactured by Satosyokuhin) immediately after the ingestion of the tea leaf powder. The period of ingesting rice by the subjects was set to 8 minutes to 10 minutes, and the subjects were subject to masticate 30 times for each bite of rice. During the ingestion of rice, the subjects were subject to ingest 100 mL of distilled water in small quantities. The subjects were prohibited from smoking, eating and drinking, and doing excessive exercises after the meal.

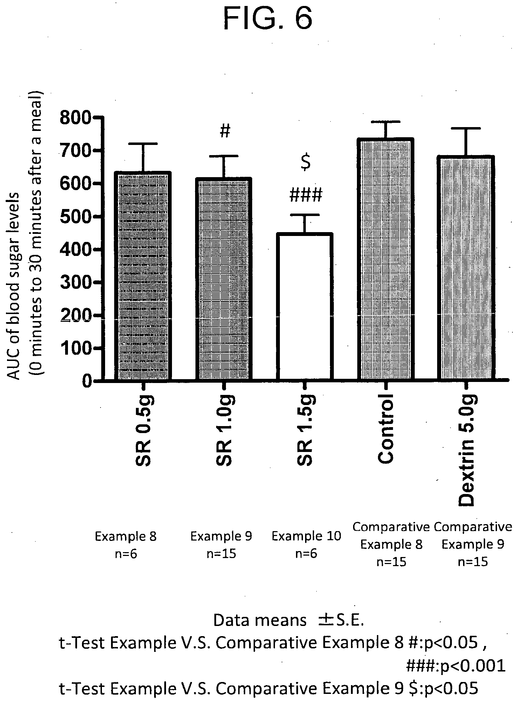

After 30 minutes have elapsed from the start of ingesting rice (set to 0 minutes), blood was collected from the fingertip of each of the subjects, and the blood sugar level was measured by the Medisafe blood sugar measurement kit (manufactured by TERUMO CORPORATION) to be a blood sugar level 30 minutes after a meal. The AUC of the blood sugar levels from 0 minutes to 30 minutes after the start of the ingestion was calculated to calculate an increase amount of the postprandial blood sugar level. The fasting blood sugar level was used for the blood sugar level 0 minutes after the start of the ingestion. The result is illustrated in FIG. 6.

[Example 9]

Example 9 was carried out in the same manner as Example 8 except that 15 persons were chosen as the subjects, and the evaluation sample of 1.0 g of the dried Sun Rouge tea leaf powder was used. The result is illustrated in FIG. 6.

[Example 10]

Example 10 was carried out in the same manner as Example 8 except that the evaluation sample of 1.5 g of the dried Sun Rouge tea leaf powder was used. The result is illustrated in FIG. 6.

[Comparative Example 8]

Comparative Example 8 was carried out in the same manner as Example 8 except that 15 persons were chosen as the subjects, and no evaluation sample was ingested. The result is illustrated in FIG. 6.

[Comparative Example 9]

Comparative Example 9 was carried out in the same manner as Example 8 except that 15 persons were chosen as the subjects, and the evaluation sample of a supplement including indigestible dextrin (product name: Kenja-no-shokutaku (TM), 6 g per packet, 5 g indigestible dextrin per packet, manufactured by Otsuka Pharmaceutical Co., Ltd., foods for specified health uses) was used. The result is illustrated in FIG. 6.

As illustrated in FIG. 6, Example 8 in which 0.5 g of the dried Sun Rouge tea leaf powder was ingested had a smaller increase in postprandial blood levels than Comparative Example 8 and Comparative Example 9. In Example 9 and Example 10 in which 1.0 g or more of the dried Sun Rouge tea leaf powder was ingested, an increase in postprandial blood sugar levels was significantly inhibited compared to Comparative Example 8, and the effect of inhibiting the increase is equal to Example 8 or more. The result of test example 6 showed that the agent of the present invention also inhibited an increase in postprandial blood sugar levels in humans, and as a result of inhibiting an increase in postprandial blood sugar levels, the agent of the present invention can prevent or ameliorate obesity, inhibit a worsening of hyperglycemia or ameliorate hyperglycemia, and exert an excellent effect of preventing or inhibiting diabetes.

Test example 7 The evaluation of an effect of promoting lipid elimination and an effect of inhibiting weight increase in mice.

Feeds with compositions similar to the ones in Example 1, Comparative Example 1 and Comparative Example 2 prepared in test example 1, the evaluation of insulin resistance, were respectively used as feed 1, feed 2 and feed 3 in the following test.

Feeds 1 to 3 have higher contents of sugar and higher contents of fat than a conventional feed which is given to mice.

[Example 11]

For the administration group of the feed 1, male mice C57BL/6J (n = 6) at the age of 12 weeks were used. After preliminary breeding the mice of the administration group for 7 days, the group was bred for 8 weeks while the mice were freely fed such that the feed 1 of each of the mice was in an amount of 4 g per day. From 6 weeks after the ingestion, the feces of each of the mice were collected for 2 weeks. The collected feces after being air-dried were samples for measuring a lipid amount.

Lipid included in the feces was measured after being extracted by Soxhlet extraction method mentioned below.

(Soxhlet extraction method)

A lipid quantitative flask was sufficiently washed and dried (105 °C for 12 hours) to determine a constant weight. 3 g of dried feces (105 °C for 12 hours) was precisely weighed and placed in cylindrical filter paper. The upper portion of the cylindrical filter paper was filled with absorbent cotton, the cylindrical filter paper was inserted into an extraction chamber of a Soxhlet extractor, and the lipid quantitative flask whose constant weight was determined was connected to the extraction chamber. 80 mL of ether was poured from the upper portion of the extraction chamber. A condenser was connected to the extractor, the extractor was placed on an electric heater, and cooling water was allowed to flow through the condenser. Ether was circulated at 60 °C to 70 °C, and the extraction was carried out for 12 hours. The cylindrical filter paper was removed from the extraction chamber, and then ether was kept being heated until all the quantity in the lipid quantitative flask was transferred into the extraction chamber. The lipid quantitative flask from which the upper portion was removed was heated in hot water bath until ether evaporated completely. After drying the lipid quantitative flask in a drying oven at 105 °C for 3 hours, and then allowing it to cool in a desiccator for 30 minutes, the lipid quantitative flask was precisely weighed. Drying, cooling, and precise weighing were repeated until a constant weight was reached.

The measurement result of a lipid amount in the feces is illustrated in Table 4.

[Comparative Example 10]

A test was carried out in the same manner as Example 11 except that the feed 2 was administered in place of the feed 1. The result is illustrated in Table 4.

[Comparative Example 11]

A test was carried out in the same manner as Example 11 except that the feed 3 was administered in place of the feed 1. The result is illustrated in Table 4.

[Table 4]

Table 4 Effect of promoting lipid elimination and effect of inhibiting weight increase in mice

| | | Example 11 (Sun Rouge group) | Comparative Example 10 (Yabukita group) | Comparative Example 11 |
|---|---|---|---|---|
| Feeds | | Feed 1 (high sugar/ high fat meal) | Feed 2 (high sugar/ high fat meal) | Feed 3 (high sugar/ high fat meal) |
| Amount of Sun Rouge added | | 1% (w/w) added | - | - |
| Amount of Yabukita added | | - | 1 % (w/w) added | - |
| Mouse weight | g (at the time of killing) | 30.2 | 31.2 | 35.6 |

(continued)

| | | Example 11 (Sun Rouge group) | Comparative Example 10 (Yabukita group) | Comparative Example 11 |
|---|---|---|---|---|
| Feeds | | Feed 1 (high sugar/ high fat meal) | Feed 2 (high sugar/ high fat meal) | Feed 3 (high sugar/ high fat meal) |
| Lipid in feces | mg/g (dried feces) | 21.4 | 19.6 | 16.2 |

The results of Table 4 demonstrate that the group of the mice in Example 11 in which the feed 1 including the Sun Rouge tea leaf extract sample was ingested had a higher proportion of lipid in the feces than the group of the mice in Comparative Example 10 in which the feed 2 including the Yabukita tea leaf extract sample was ingested or the group of the mice in Comparative Example 11 in which the feed 3 of a blank including no evaluation sample was injected, and the Sun Rouge tea leaf extract included in the feed 1 had an excellent effect of promoting lipid elimination. It is also illustrated that the group of the mice in Example 11 in which the feed 1 was ingested had the lowest weight at the time of killing compared to the group of the mice in Comparative Example 10 in which the feed 2 was ingested or the group of the mice in Comparative Example 11 in which the feed 3 was ingested, and the Sun Rouge tea leaf extract included in the feed 1 had an excellent effect of inhibiting a weight increase.

The results mentioned above demonstrate that the agent of the present invention exerts an excellent effect of promoting lipid elimination and an excellent effect of inhibiting a weight increase, can prevent or ameliorate obesity, can inhibit a worsening of hyperglycemia or ameliorate hyperglycemia, and can exert an excellent effect of preventing or inhibiting diabetes.

Test example 8 The evaluation of an effect of inhibiting lipolysis

[Example 12]

1.0 mL of distilled water was added to 20 mg of dried Sun Rouge tea leaf powder to extract components at the temperature of 100 °C for 10 minutes. The mixture was centrifuged at 1700 g for 10 minutes, and then filtered using a 0.2 $\mu$m filter (manufactured by Whatman) to obtain an extract of the dried Sun Rouge tea leaves. The dried tea leaf powder was prepared by pulverizing unprocessed tea leaves by the multi-beads shocker (manufactured by Yasui Kikai Corporation).

The lipase derived from pig pancreas (manufactured by Sigma-Aldrich Co. LLC) was prepared such that the final concentration was 102 U/L. "A: the lipase activity of the mixed solution of the extract of the dried Sun Rouge tea leaves and the lipase solution" and "B: the lipase activity of the lipase solution" were respectively measured by the Lipase Kit S (manufactured by DS Pharma Biomedical Co., Ltd.), and the inhibition rate of the lipase activity of the extract of the dried Sun Rouge tea leaves was calculated by dividing A by B. The result is illustrated in Table 5.

[Comparative Example 12]

Comparative Example 12 was carried out in the same manner as Example 12 except that dried Yabukita tea leaves were used in place of the dried Sun Rouge tea leaves. The result is illustrated in Table 5.

[Table 5]

Table 5 Inhibition rates of pancreatic lipase activity

| | Tea leaves | Inhibition rates of lipase activity |
|---|---|---|
| Example 12 | Sun Rouge | 79.1% |
| Comparative Example 12 | Yabukita | 44.5% |

Table 5 illustrates that Example 12 in which the extract sample of the dried Sun Rouge tea leaf powder was used showed a higher effect of inhibiting lipase activity than Comparative Example 12 in which the extract sample of the dried Yabukita tea leaf powder was used, and had an excellent effect of inhibiting lipolysis.

Test example 9 The evaluation of an effect of enhancing lipid metabolism

[Example 13]

The liver of each of the mice killed in Example 1 of text example 1 was extracted to perform a microarray analysis (Whole Mouse Genome Oligo DNA Microarray Kit Ver. 2.0 manufactured by Agilent Technologies).

For comparison, the liver of each of the mice killed in Comparative Example 2 of text example 1 was extracted to perform a microarray analysis.

For the resulting data of gene expression amounts, the genes whose expression amount was 2.0 or more in Example 1 and Comparative Example 2 were extracted as up-regulated genes.

Functional Annotation analysis of DAVID (http://david.abcc.ncifcrf.gov/home.jsp) was performed on Entrez Gene ID of the extracted genes, and then pathways significantly including the up-regulated genes were extracted from KEGG-PATHWAY. The results are illustrated in Table 6.

[Comparative Example 13]

Comparative Example 13 was carried out in the same manner as Example 13 except that the mice of Example 1 used in Example 13 were changed to the mice in Comparative Example 1. The results are illustrated in Table 6.

[Table 6]

Table 6

| Go term | Example 13 Sun Rouge group / High calorie group | Comparative Example 13 Yabukita group / High calorie group |
|---|---|---|
| Steroid hormone biosynthesis | 1.30E-04 | - |
| Primary bile acid biosynthesis | 2.80E-02 | - |
| PPAR signaling pathway | 5.10E-02 | - |
| Adipocytokine signaling pathway | 6.50E-03 | 9.00E-02 |

The results of Table 6 demonstrate that the mice that ingested the feed including the Sun Rouge tea leaf extract sample had improved metabolisms in any of the lipid metabolism pathways illustrated in Table 6.

From the evaluation results of test example 8 and text example 9, the Sun Rouge tea leaf extract sample presumably has an excellent effect of promoting lipid elimination due to the effect of inhibiting lipolysis and the effect of enhancing lipid metabolism.

Text example 10 The evaluation of the thermal stability of tea leaf extract

[Example 14]

Tea leaf extract was obtained under the following conditions.

•Tea leaves: Tea leaves obtained by picking "Sun Rouge" tea leaves of the year 2012, and then steaming and drying

- Pretreatment: Pulverization (average particle diameter: 309 $\mu$m) by the multi-beads shocker (TM, Yasui Kikai Corporation)

- Temperature: 70 °C

- Period: 10 minutes

- Extraction solvent and the quantity thereof: Distilled water (DW) in an amount of 50 times more than the dry weight of the tea leaves

The amount of delphinidin or its glycosides, and the amount of catechins relative to the dry weight of the resulting tea leaf extract were analyzed by HPLC under the conditions mentioned in the literature (Mari Maeda-Yamamoto et al. Chemical analysis and acetylcholinesterase inhibitory effect of anthocyanin-rich red leaf tea (cv. Sunrouge). J Sci Food Agric 92:2379-2386(2012).), and were 0.2% by weight and 18.5% by weight respectively.

The tea leaf extract was concentrated, and then pulverized by the Spray Dryer (product name: Pulvis Mini-Spray GA-32 manufactured by Yamato Scientific Co., Ltd.) to prepare tea leaf extract powder. This tea leaf extract powder was heated at the temperature of 100 °C for 120 minutes to measure the residual rate of delphinidin after the heat treatment.

[Comparative Example 14]

The specification value of the extract powder derived from maqui berry (product name: maqui berry extract manufactured by ORYZA OIL & FAT CHEMICAL CO., LTD.) which is an already distributed product rich in delphinidin was used.

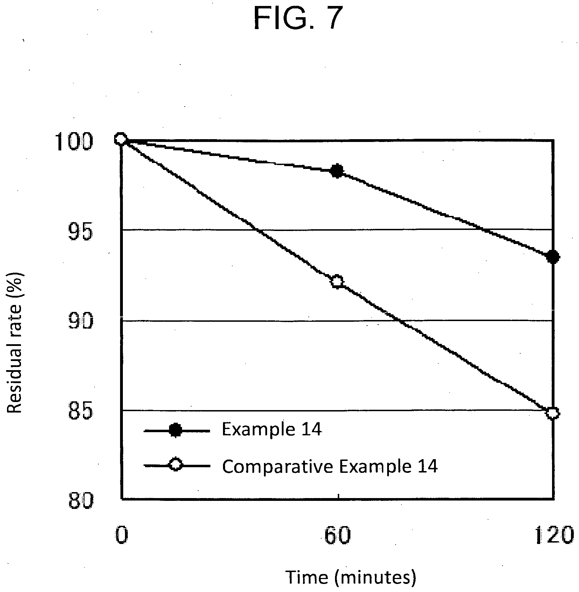

The results of Example 14 and Comparative Example 14 are illustrated in FIG. 7.

The thermal stability of the tea leaf extract powder (Example 14) was superior to the thermal stability of the maqui berry extract powder including delphinidin (Comparative Example 14).

**Claims**

1. An agent for preventing or ameliorating diabetes, comprising a component of a hybrid plant of Camellia sinensis and Camellia taliensis as an active ingredient.

2. An agent for preventing or ameliorating metabolic syndrome, comprising a component of a hybrid plant of Camellia sinensis and Camellia taliensis as an active ingredient.

3. An agent for inhibiting or ameliorating an increase in HOMA-IR, comprising a component of a hybrid plant of Camellia sinensis and Camellia taliensis as an active ingredient.

4. An agent for inhibiting or ameliorating fat accumulation, comprising a component of a hybrid plant of Camellia sinensis and Camellia taliensis as an active ingredient.

5. An agent for inhibiting an increase in a postprandial blood sugar level, comprising a component of a hybrid plant of Camellia sinensis and Camellia taliensis as an active ingredient.

6. An agent for inhibiting glycolysis, comprising a component of a hybrid plant of Camellia sinensis and Camellia taliensis as an active ingredient.

7. An agent for inhibiting $\alpha$-glucosidase activity, comprising a component of a hybrid plant of Camellia sinensis and Camellia taliensis as an active ingredient.

8. An agent for inhibiting amylase activity, comprising a component of a hybrid plant of Camellia sinensis and Camellia taliensis as an active ingredient.

9. An agent for promoting lipid elimination, comprising a component of a hybrid plant of Camellia sinensis and Camellia taliensis as an active ingredient.

10. An agent for inhibiting lipase activity and enhancing lipid metabolism, comprising a component of a hybrid plant of Camellia sinensis and Camellia taliensis as an active ingredient.

11. The agent according to any one of claims 1 to 10, wherein the hybrid plant of Camellia sinensis and Camellia taliensis is Sun Rouge (Camellia sinensis (L.) Kuntze sp Sunrouge).

12. The agent according to any one of claims 1 to 11, comprising a tea leaf extract of the hybrid plant of Camellia sinensis and Camellia taliensis.

13. The agent according to claim 12, wherein the tea leaf extract comprises 0.13% by weight or more of delphinidin or its glycoside relative to the dry weight of the tea leaf extract.

14. A method of ameliorating or preventing diabetes, comprising ameliorating or preventing diabetes of a subject by using an agent or a composition comprising a component of a hybrid plant of Camellia sinensis and Camellia taliensis as an active ingredient to the subject.

# FIG. 1

P<0.001

P<0.01

n.s.

Fat weight around testicles (g)

| Sunrouge | Yabukita | Control |
|---|---|---|
| Example 1 | Comparative Example 1 | Comparative Example 2 |

Data means ±S.E. , n=6 , Tukey's Test

# FIG. 2

Data means ±S.E. , n=6 , Tukey's Test

# FIG. 3

# FIG. 4

P<0.01

n.s.

p<0.001

|  | Sunrouge | Yabukita | Control |
|---|---|---|---|

AUC of blood sugar levels
(0 minutes to 30 minutes after a meal)

Sunrouge — Example 2

Yabukita — Comparative Example 3

Control — Comparative Example 4

Data means ±S.E. , n=6 , Tukey's Test

# FIG. 5

P<0.001

Inhibition rate of α-glucosidase activity (%)

| | |
|---|---|
| **Sunrouge** | **Yabukita** |
| Example 3 | Comparative Example 5 |

Data means ±S.E. , n=3 , t-Test

# FIG. 6

Data means ±S.E.
t-Test Example V.S. Comparative Example 8 #:p<0.05 ,
###:p<0.001
t-Test Example V.S. Comparative Example 9 $:p<0.05

# FIG. 7

## INTERNATIONAL SEARCH REPORT

International application No.

PCT/JP2015/058488

### A. CLASSIFICATION OF SUBJECT MATTER

*A61K36/82*(2006.01)i, *A61K31/352*(2006.01)i, *A61K31/353*(2006.01)i, *A61P3/06* (2006.01)i, *A61P3/10*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K36/82, A61K31/352, A61K31/353, A61P3/06, A61P3/10, A61P43/00

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 3-133928 A (Mitsui Norin Co., Ltd.),<br>07 June 1991 (07.06.1991),<br>claims; examples<br>& US 5318986 A      & EP 423419 A1<br>& KR 10-0178522 B | 1-11<br>1-13 |
| X<br>Y | JP 2002-326932 A (Kao Corp.),<br>15 November 2002 (15.11.2002),<br>claims<br>(Family: none) | 1-11<br>1-13 |
| X<br>Y | JP 2007-43907 A (Fukujuen Co., Ltd.),<br>22 April 2007 (22.04.2007),<br>paragraphs [0003] to [0007]<br>(Family: none) | 1-11<br>1-13 |

[X] Further documents are listed in the continuation of Box C.          [ ] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 June 2015 (12.06.15) | 23 June 2015 (23.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

| International application No. |
|---|
| PCT/JP2015/058488 |

C (Continuation).  DOCUMENTS CONSIDERED TO BE RELEVANT

| Category* | Citation of document, with indication, where appropriate, of the relevant passages | Relevant to claim No. |
|---|---|---|
| X<br>Y | JP 52-116473 A  (Inverni Della Beffa S.p.A.),<br>29 September 1977 (29.09.1977),<br>claims; examples<br>& US 4166861 A           & GB 1575004 A<br>& DE 2711927 A | 1-11<br>1-13 |
| X<br>Y | JP 60-156614 A  (Mitsui Norin Co., Ltd.),<br>16 August 1985 (16.08.1985),<br>claims; examples<br>(Family: none) | 1-11<br>1-13 |
| X<br>Y | JP 1-299224 A  (Ito En, Ltd.),<br>04 December 1989 (04.12.1989),<br>claims; examples<br>(Family: none) | 1-11<br>1-13 |
| X<br>Y | JP 2005-247747 A  (Ito En, Ltd.),<br>15 September 2005 (15.09.2005),<br>claims; examples<br>(Family: none) | 1-11<br>1-13 |
| P,X<br>P,Y | Process Biochem., 2014.06, Vol.49, pp.1457-1463,<br>title, abstract, tables 1, 2 | 1-11<br>1-13 |
| X<br>Y | Int. J. Mol. Sci., 2010, Vol.11, pp.3387-3396,<br>title, abstract, table 1 | 1-11<br>1-13 |
| X<br>Y | J. Agric. Food Chem., 2005, Vol.53, pp.28-31,<br>title, abstract, fig. 2, 3, compounds 1 to 3, 6 | 1-11<br>1-13 |
| X<br>Y | Nutrition, 2012, Vol.28, pp.1055-1062, title,<br>abstract, each table, each figure | 1-11<br>1-13 |
| X<br>Y | Toxicology, 1981, Vol.19, pp.151-158, Fig.5 | 1-11<br>1-13 |
| X<br>Y | JP 10-330264 A  (Takeda Food Products, Ltd.),<br>15 December 1998 (15.12.1998),<br>each example<br>(Family: none) | 1-11<br>1-13 |
| Y | J. Agric. Food Chem., 2011, Vol.59, pp.4779-4782, title, abstract, page 4779, right column to the next page, left column, column of 'Extraction and Isolation of-', fig. 1, 2, tables 1, 2 | 1-13 |
| X<br>Y | WO 2011/108487 A1  (Kyushu University),<br>09 September 2011 (09.09.2011),<br>claims; paragraphs [0027], [0040], [0073]<br>& JP 2013-126951 A | 1-13<br>1-13 |

Form PCT/ISA/210 (continuation of second sheet) (July 2009)

**INTERNATIONAL SEARCH REPORT**

International application No.

PCT/JP2015/058488

C (Continuation).    DOCUMENTS CONSIDERED TO BE RELEVANT

| Category* | Citation of document, with indication, where appropriate, of the relevant passages | Relevant to claim No. |
|---|---|---|
| Y | J. Sci. Food Agric., 2012, Vol.92, pp.2379-2386, title, abstract, page 2380, left column, column of 'Tea extracts', fig. 1, tables 1, 2 | 1-13 |

Form PCT/ISA/210 (continuation of second sheet) (July 2009)

<div align="center">

**INTERNATIONAL SEARCH REPORT**

</div>

| International application No. |
|---|
| PCT/JP2015/058488 |

| **Box No. II**      **Observations where certain claims were found unsearchable (Continuation of item 2 of first sheet)** |
|---|

This international search report has not been established in respect of certain claims under Article 17(2)(a) for the following reasons:

1. ☒ Claims Nos.: 14
    because they relate to subject matter not required to be searched by this Authority, namely:
    The invention of claim 14 involves "a method for treatment of the human body or animal body by surgery or therapy and a method for diagnosis of the human body or animal body".

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

| **Box No. III**      **Observations where unity of invention is lacking (Continuation of item 3 of first sheet)** |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

                                     ☐ The additional search fees were accompanied by the applicant's protest but the applicable protest fee was not paid within the time limit specified in the invitation.

                                       ☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6036746 B
- JP H759539 B
- JP S60156614 B
- JP 2002326932 A

**Non-patent literature cited in the description**

- *Journal of Nutritional Food,* 1999, vol. 2 (4
- **MARI MAEDA-YAMAMOTO et al.** Chemical analysis and acetylcholinesterase inhibitory effect of anthocyanin-rich red leaf tea (cv. Sunrouge). *J Sci Food Agric,* 2012, vol. 92, 2379-2386